# EUROPEAN PATENT SPECIFICATION

(11) **EP 3 915 566 B1**
(45) Date of publication and mention of the grant of the patent: **08.11.2023**
(21) Application number: 20745833.2
(22) Date of filing: 23.01.2020
(51) Int. Cl.: A61K 31/575, A61P 1/00, A61P 29/00, A23L 33/10

(54) **COMPOSITION FOR PREVENTING OR TREATING INFLAMMATORY BOWEL DISEASE, CONTAINING, AS ACTIVE INGREDIENT, TAURODEOXYCHOLIC ACID OR PHARMACEUTICALLY ACCEPTABLE SALT THEREOF**
ZUSAMMENSETZUNG ZUR VORBEUGUNG ODER BEHANDLUNG VON ENTZÜNDLICHEN DARMERKRANKUNGEN MIT TAURODESOXYCHOLSÄURE ODER EINEM PHARMAZEUTISCH VERTRÄGLICHEN SALZ DAVON ALS WIRKSTOFF
COMPOSITION POUR PRÉVENIR OU TRAITER UNE MALADIE INTESTINALE INFLAMMATOIRE, CONTENANT, EN TANT QUE PRINCIPE ACTIF, DE L'ACIDE TAURODÉSOXYCHOLIQUE OU UN SEL PHARMACEUTIQUEMENT ACCEPTABLE DE CE DERNIER

(30) Priority: 23.01.2019 KR 20190008527
(43) Date of publication of application: 01.12.2021
(73) Proprietor: SHAPERON Inc., Seoul 06373 (KR)
(72) Inventor: SEONG, Seung-Yong, Seoul 03080 (KR); SEO, Sang-Uk, Seoul 03080 (KR); MUNKHBILEG, Bolormaa, Seoul 03080 (KR)
(74) Representative: Vossius & Partner Patentanwälte Rechtsanwälte mbB
(86) International application number: PCT/KR2020/001179
(87) International publication number: WO 2020/153794

(56) References cited:
- US-A1- 2017 196 895
- VAN DEN BOSSCHE LIEN ET AL: "Ursodeoxycholic Acid and Its Taurine- or Glycine-Conjugated Species Reduce Colitogenic Dysbiosis and Equally Suppress Experimental Colitis in Mice", APPLIED AND ENVIRONMENTAL MICROBIOLOGY, [Online] vol. 83, no. 7, 1 April 2017 (2017-04-01), XP055950590, US ISSN: 0099-2240, DOI: 10.1128/AEM.02766-16 Retrieved from the Internet: URL:https://journals.asm.org/doi/pdf/10.11 28/AEM.02766-16> [retrieved on 2022-08-09]
- CHANG SOOGHEE ET AL: "Taurodeoxycholate Increases the Number of Myeloid-Derived Suppressor Cells That Ameliorate Sepsis in Mice", FRONTIERS IN IMMUNOLOGY, vol. 9, 1 January 2018 (2018-01-01), XP055950574, DOI: 10.3389/fimmu.2018.01984
- Jiao He; Jinru Liang; Sha Zhu; Wenna Zhao; Yongmin Zhang; Wenji Sun;: "Protective effect of taurohyodeoxycholic acid from Pulvis Fellis Suis on trinitrobenzene sulfonic acid induced ulcerative colitis in mice", European journal of pharmacology, vol. 670, no. 1, 27 August 2011 (2011-08-27), pages 229-235, XP028312072, ISSN: 0014-2999, DOI: 10.1016/j.ejphar.2011.08.036
- Yang Yang; He Jiao; Suo Yuan; Lv Le; Wang Jingjing; Huo Chuanchuan; Zheng Zongwei; Wang Ziye; Li Jing; Sun Wenji; Zhang Yongmin: "Anti-inflammatory effect of taurocholate on TNBS-induced ulcerative colitis in mice", Biomedicine & Pharmacotherapy, vol. 81, 28 April 2016 (2016-04-28), pages 424-430, XP029566187, ISSN: 0753-3322, DOI: 10.1016/j.biopha.2016.04.037
- Mühlbauer M., Allard B., Bosserhoff A. K., Kiessling S., Herfarth H., Rogler G., Schölmerich J., Jobin C., Hellerbrand C.: "Differential effects of deoxycholic acid and taurodeoxycholic acid on NF-kappaB signal transduction and IL -8 gene expression in colonic epithelial cells", American Journal of Physiology- Gastrointestinal and Liver Physiology, vol. 286, no. 6, 15 January 2003 (2003-01-15), pages G1000-G1008, XP055804674, ISSN: 0193-1857, DOI: 10.1152/ajpgi.00338.2003
- Lien Van Den Bossche, Daniel Borsboom, Sarah Devriese, Sophie Van Welden, Tom Holvoet, Lindsey Devisscher, Pieter Hindryckx, Marti: "Tauroursodeoxycholic acid protects bile acid homeostasis under inflammatory conditions and dampens Crohn ' s disease-like ileitis", Laboratory Investigation, vol. 97, no. 5, 1 May 2017 (2017-05-01), pages 519-529, XP055726994, ISSN: 0023-6837, DOI: 10.1038/labinvest.2017.6

## Description

### [Technical Field]

The present invention relates to a composition for use in the prevention or treatment of inflammatory bowel disease or ischemic colitis, which contains taurodeoxycholic acid or a pharmaceutically acceptable salt thereof as an active ingredient, more specifically to a pharmaceutical composition or a health food for use in the prevention or treatment of inflammatory bowel disease or ischemic colitis, which contains taurodeoxycholic acid or a pharmaceutically acceptable salt thereof as an active ingredient.

### [Background Art]

Inflammatory bowel disease is a disease causing chronic and unexplained inflammation in the bowels and can be clinically classified into ulcerative colitis and Crohn's disease.

Ulcerative colitis is a disease in which sores (erosion) or persistent inflammation is continuously formed in the mucous membrane of the large bowel and ulcers that affect the mucosal epithelium and lamina propria of the colon and rectum are continuously formed. 95% of ulcerative colitis affects the rectum and spreads continuously and circumferentially to the closer part of the large bowel. Symptoms of ulcerative colitis include abdominal pain, bloody stool, spots, and diarrhea, and systemic symptoms such as fever, body weight loss, and anemia are caused in severe cases. Ulcerative colitis develops from teens to young adults, occurs at all ages, and affects men and women equally. In recent years, as the incidence rate of ulcerative colitis has increased worldwide, the importance thereof has increased. The incidence rate of ulcerative colitis is the highest in the Western world and is estimated to be 30 per 100,000 people. According to the Asia-Pacific Crohn and Colitis epidemiological study, the incidence rate of ulcerative colitis in Asia and the Middle East was 6.3 per 100,000 people on the average in 2012, and this means that the incidence rate of ulcerative colitis in Asia and the Middle East has increased dramatically. In Korea, the incidence rate of ulcerative colitis has increased from 0.22 per 100,000 people in 1886 to 3.62 per 100,000 people after 2005. Ulcerative colitis is a chronic disease that affects the quality of life, is a disease that is financially expensive, and may lead to death if not treated properly.

Crohn's disease is a disease in which lesions such as ulcers are discontinuously formed in any part of the digestive tract from the mouth to the anus, and symptoms such as fever, body weight loss, general malaise, and anemia are caused in severe cases in addition to abdominal pain, diarrhea, and bloody stool.

The cause or pathophysiology of such inflammatory bowel disease is not yet clearly known and thus the fundamental treatment method of inflammatory bowel disease has not been established. Hence, the treatment of inflammatory bowel disease is not aimed at complete treatment, but drugs which delay and alleviate the progression of symptoms and maintain this condition for as long as possible are used. As drugs for such popular therapy, mainly aminosalicylic acid preparations, adrenal cortical steroids, immunosuppressants, TNF-α monoclonal antibodies, and the like are used, but various side effects have been reported. For example, sulfasalazine that is frequently used as an aminosalicylic acid preparation, has been reported to have side effects such as nausea, vomiting, loss of appetite, rash, headache, liver injury, leukocytopenia, abnormal red blood cells, proteinuria, and diarrhea. Prednisolone that is an adrenal cortical steroid is used by oral administration, enema, suppository, intravenous injection and the like but has strong side effects such as gastric ulcer or necrosis of the femoral head due to long-term use. Biological therapeutic agents such as TNF-α monoclonal antibodies have advantages of predicting, preventing, and treating complications in patients, restoring nutritional deficiencies, and reducing mortality but have problems such as high cost and susceptibility to infection in some patients, side effects, and occurrence of low-responders. Hence, in the treatment of inflammatory bowel disease, a new therapeutic agent with a high efficiency but few side effects is required.

Meanwhile, bile acid is an important physiological molecule in the secretion of bile for absorption of lipids, harmful metabolites, and nutrients in the bowels and is produced in perivenous hepatocytes of human liver. The primary bile acids formed in the human body are chenodeoxycholic acid and cholic acid. Chenodeoxycholic acid is conjugated with taurine or glycine to produce a total of eight conjugated bile acids such as taurochenodeoxycholate and glycochenodeoxycholate. Deoxycholic acid, lithocholic acid, ursodeoxycholic acid and the like are formed as secondary bile acids by the influence of intestinal microbes on the primary bile acids. Cholic acid is converted to deoxycholic acid and chenodeoxycholic acid is converted to lithocolinic acid or ursodeoxycholic acid. Taurine is conjugated to deoxycholic acid to form taurodeoxycholic acid. These bile acids are known to play an important role as signaling molecules in the absorption of dietary lipids and in the regulation of cholesterol homeostasis and systemic endocrine function and to regulate immune homeostasis, intestinal circulation, and metabolism by activating various signal transduction pathways, and are thus applied as therapeutic agents. For example, ursodeoxycholic acid that is present in a significantly small amount as a secondary bile acid has proven its pharmacological effect in improvement of the liver function and is applied as a therapeutic agent for liver disease. Van den Bossche et al., report that ursodeoxycholic acid and its taurine- or glycine-conjugated species reduce experimental colitis in mice.

Accordingly, the present inventors have tried to develop a new therapeutic agent which exhibits excellent therapeutic effect on inflammatory bowel disease, is safe, and has few side effects and, as a result, confirmed that the clinical symptoms and histopathological symptoms caused by inflammatory bowel disease are alleviated and the increase in inflammatory cells in bowels and the production of pro-inflammatory cytokines are suppressed by using a salt of taurodeoxycholic acid, found that taurodeoxycholic acid and a pharmaceutically acceptable salt thereof can be used as an active ingredient of a composition for prevention or treatment of inflammatory bowel disease, and thus completed the present invention.

### [Citation List]

### [Non Patent Literature]

Silvio D., Claudio F., 2011, Ulcerative Colitis, New England Journal of Medicine 2011., 365:1713-25
Kaplan, G.G., 2015. The global burden of IBD: from 2015 to 2025. Nature reviews. Gastroenterology & Hepatology, 12(12), pp.720-7.
Kirsner, J. B. Historical aspects of inflammatory bowel disease. J. Clinical Gastroenterology. 10, 286-297 (1988).
Geremia, A. et al., 2014. Autoimmunity Reviews Innate and adaptive immunity in inflammatory bowel disease. Autoimmunity Reviews, 13(1), pp.3-10.
Fournier, B.M. & Parkos, C.A., 2012. The role of neutrophils during intestinal inflammation., 5(4), pp.354-366.
Baars, A. et al., 2015. The Gut Microbiota as a Therapeutic Target in IBD and Metabolic Disease: A Role for the Bile Acid Receptors FXR and TGR5. , pp.641-666.
Strober W, Fuss IJ. 2011 Proinflammatory cytokines in the pathogenesis of inflammatory bowel diseases. Gastroenterology 2011; 140:1756-1767. doi: 10.1053/j.gastro.2011.02.016 PMID: 21530742.
Klein, A. & Eliakim, R., 2010. Nonsteroidal anti-inflammatory drugs and inflammatory bowel disease. Pharmaceuticals, 3(4), pp.1084-1092.
Sales-Campos, H. et al., 2015. Classical and recent advances in the treatment of inflammatory bowel diseases. Brazilian journal of medical and biological research, 48(2), pp.96-107.
Russell DW 2003. "The enzymes, regulation, and genetics of bile acid synthesis". Annu. Rev. Biochem. 72: 137-74.
Van den Bossche Lien et al. 2017. Ursodeoxycholic acid and its taurine- or glycine-conjugated species reduce colitogenic dysbiosis and equally suppress experimental colitis in mice. Applied and Environmental microbiology. 83(7)DOI: 10.1128/AEM.02766-16

### [Summary of Invention]

### [Technical Problem]

An object of the present invention is to provide a composition for use in the prevention, treatment, or improvement of inflammatory bowel disease or ischemic colitis, which contains taurodeoxycholic acid or a pharmaceutically acceptable salt thereof as an active ingredient.

### [Solution to Problem]

In order to achieve the objects of the present invention, the present invention provides a pharmaceutical composition for use in the prevention or treatment of inflammatory bowel disease or ischemic colitis, which contains taurodeoxycholic acid or a pharmaceutically acceptable salt thereof as an active ingredient.

The present invention also provides a health food for use in the prevention or improvement of inflammatory bowel disease or ischemic colitis, which contains taurodeoxycholic acid or a pharmaceutically acceptable salt thereof as an active ingredient.

### [Advantageous Effects of Invention]

The present inventors have confirmed that the clinical symptoms and histopathological symptoms caused by inflammatory bowel disease are alleviated and the increase in inflammatory cells in bowels and the production of pro-inflammatory cytokines are suppressed by using a salt of taurodeoxycholic acid, and thus taurodeoxycholic acid and a pharmaceutically acceptable salt thereof can be used as an active ingredient of a composition for prevention or treatment of inflammatory bowel disease.

### [Brief Description of Drawings]

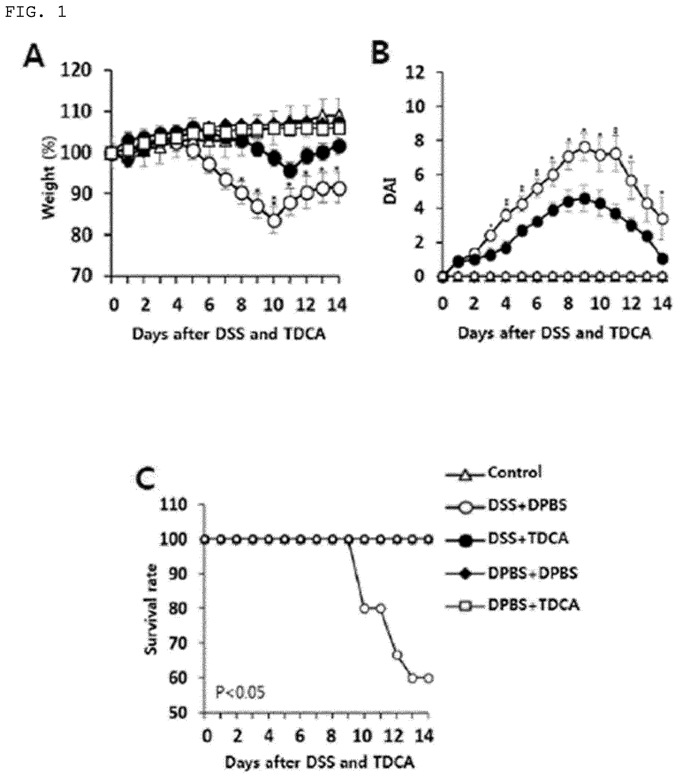

FIG. 1 is a diagram illustrating the clinical symptoms-alleviating effect by sodium taurodeoxycholic acid (TDCA) in a mouse model in which inflammatory bowel disease is induced by administration of dextran sodium sulfate (DSS), FIG. 1A illustrates the body weight change, FIG. 1B illustrates the disease activity index (DAI) change, and FIG. 1C illustrates the survival rate change (*p < 0.05, **p < 0.01, ***p < 0.001).

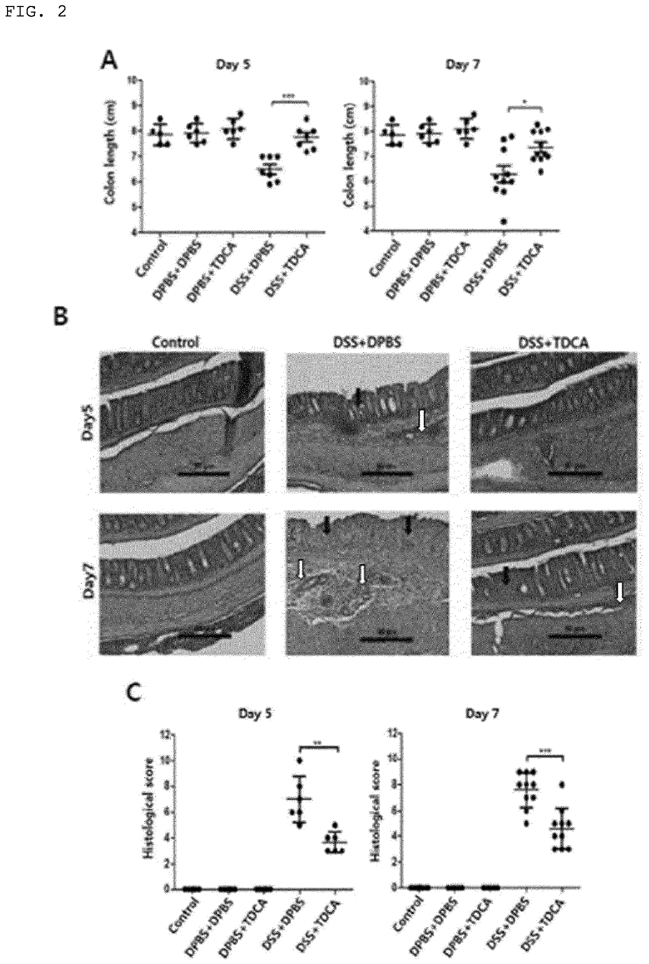

FIG. 2 is a diagram illustrating the histopathological symptoms alleviating-effect by TDCA in a mouse model in which inflammatory bowel disease is induced by administration of DSS, FIG. 2A illustrates the colon length change, FIG. 2B illustrates the histological state change, and FIG. 2C illustrates the histological score change (red arrow: inflammatory cell infiltration, black arrow: mucosal epithelium and intestinal crypt damage).

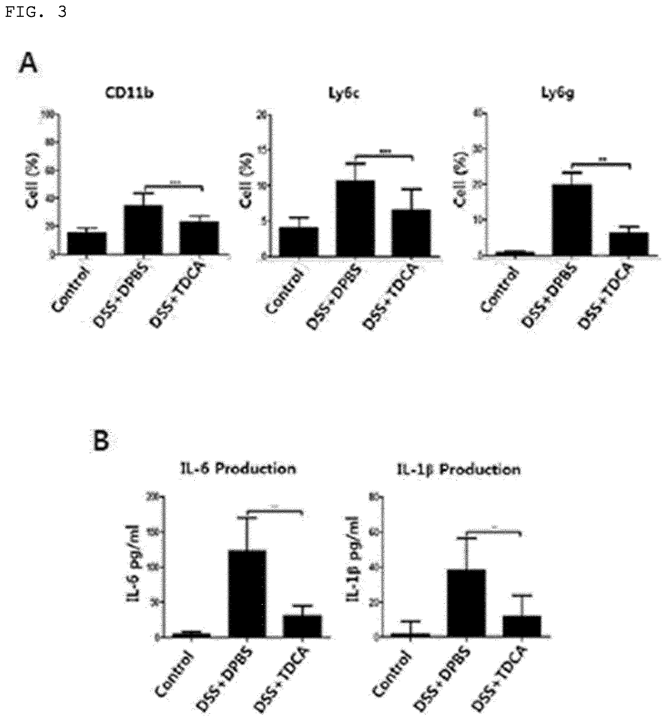

FIG. 3 is a diagram illustrating the effect of decreasing inflammatory cells and pro-inflammatory cytokines in the bowels by TDCA in a mouse model in which inflammatory bowel disease is induced by administration of DSS, FIG. 3A illustrates the changes in inflammatory cells, and FIG. 3B illustrates the changes in pro-inflammatory cytokines.

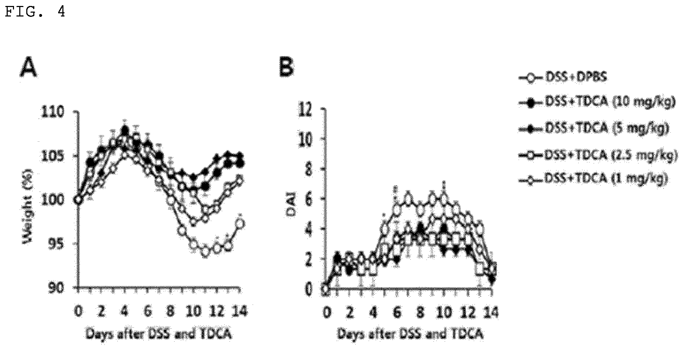

FIG. 4 is a diagram illustrating the clinical symptoms-alleviating effect depending on the TDCA concentration in a mouse model in which inflammatory bowel disease is induced by administration of DSS, FIG. 4A illustrates the body weight change, and FIG. 4B illustrates the DAI change.

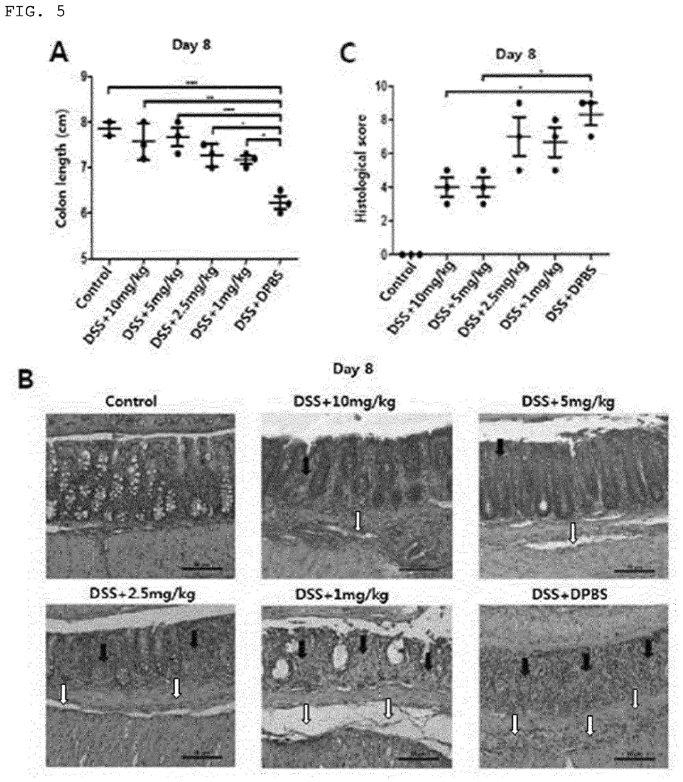

FIG. 5 is a diagram illustrating the histopathological symptoms-alleviating effect depending on the TDCA concentration in a mouse model in which inflammatory bowel disease is induced by administration of DSS, FIG. 5A illustrates the colon length change, FIG. 5B illustrates the histological state change, and FIG. 5C illustrates the histological score change.

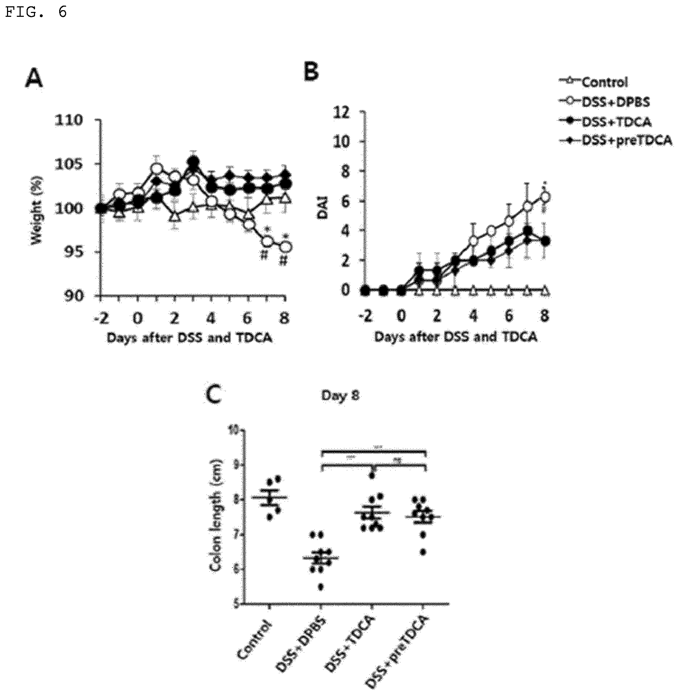

FIG. 6 is a diagram illustrating the clinical symptoms and pathological symptoms-alleviating effect by TDCA administration before or after DSS administration in a mouse model in which inflammatory bowel disease is induced by administration of DSS, FIG. 6A illustrates the body weight change, FIG. 6B illustrates the DAI change, and FIG. 6C illustrates the colon length change.

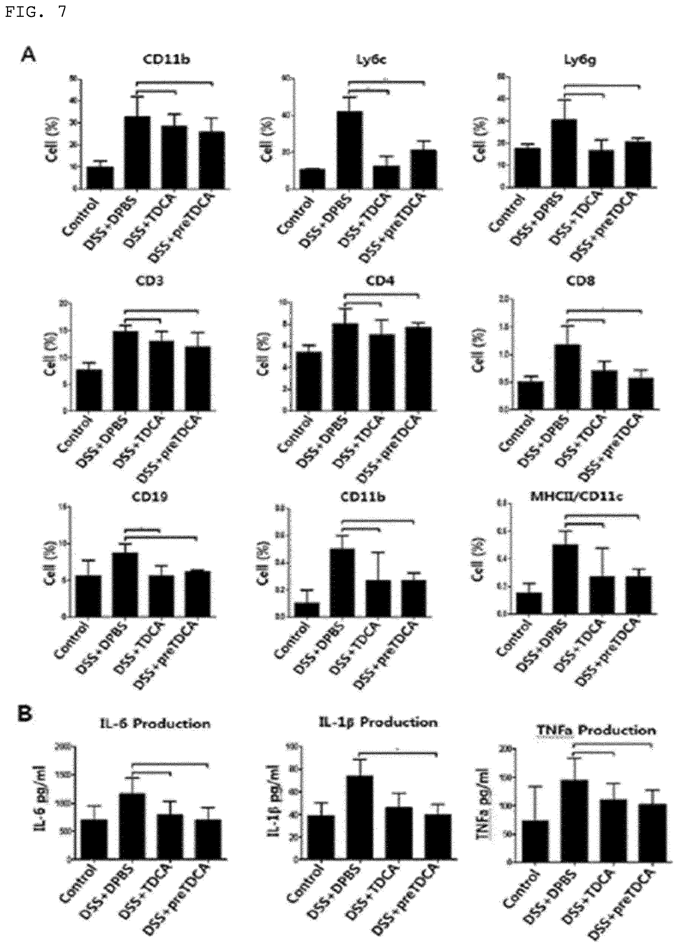

FIG. 7 is a diagram illustrating the effect of decreasing inflammatory cells and pro-inflammatory cytokines in the bowels by TDCA administration before or after DSS administration in a mouse model in which inflammatory bowel disease is induced by administration of DSS, FIG. 7A illustrates the changes in inflammatory cells, and FIG. 7B illustrates the changes in pro-inflammatory cytokines.

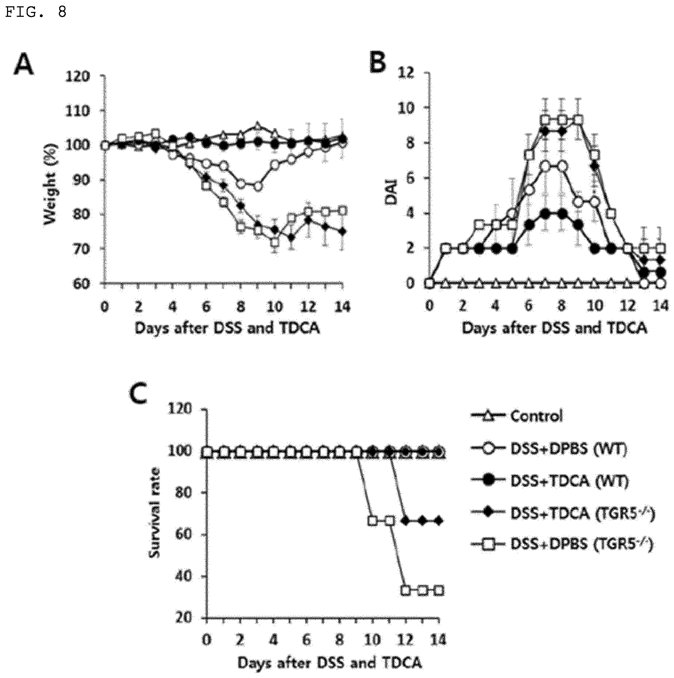

FIG. 8 is a diagram illustrating the changes in clinical symptoms by TDCA in a TGR5 knock-out (TGR5^{-/-}) mouse model in which inflammatory bowel disease is induced by administration of DSS, FIG. 8A illustrates the body weight change, FIG. 8B illustrates the DAI change, and FIG. 8C illustrates the survival rate change.

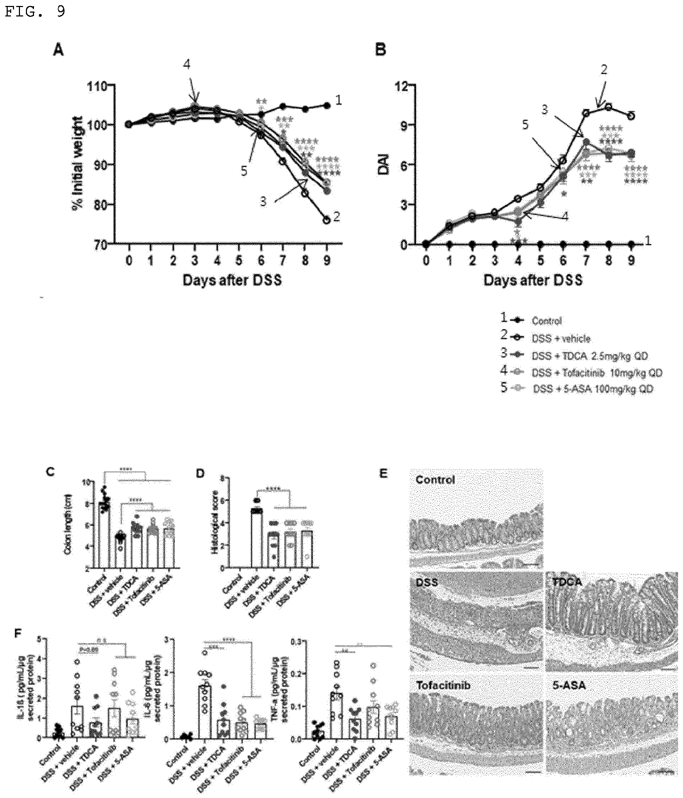

FIG. 9 is a diagram illustrating the changes in clinical symptoms in a mouse model in which inflammatory bowel disease is induced by administration of DSS in comparison with those by prescription drugs, tofacitinib and 5-ASA (5-acetyl salicylic acid) used in the clinic, FIG. 9A illustrates the body weight change, FIG. 9B illustrates the DAI change, FIG. 9C illustrates the colon length change, FIG. 9D illustrates the changes in lesion index of colon tissue, FIG. 9E illustrates the changes in pro-inflammatory cytokines in colon tissue, and FIG. 9F illustrates the representative lesion changes of colon tissue in respective administration groups.

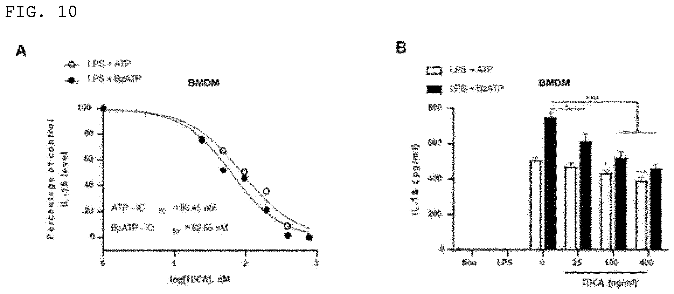

FIG. 10 is a diagram illustrating the inhibitory ability of TDCA on the secretion of pro-inflammatory cytokines that are important for inducing ulcerative colitis confirmed by isolating and culturing bone marrow-derived macrophages from mice, FIG. 10A illustrates the percentage changes in inhibitory ability depending on the TDCA concentration, and FIG. 10B illustrates the changes in secretion amount of pro-inflammatory cytokine IL-1β depending on the TDCA concentration.

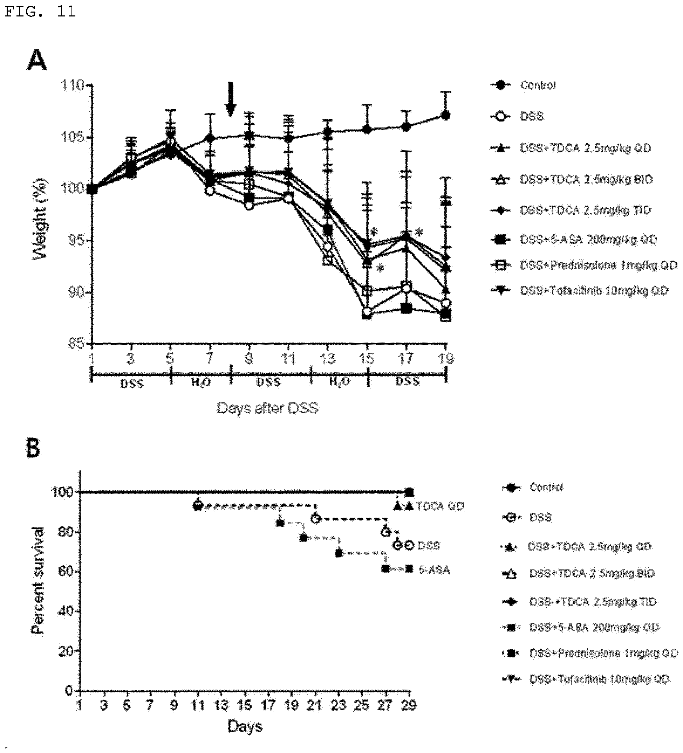

FIG. 11 is a diagram illustrating the changes in clinical symptoms in a mouse model in which chronic inflammatory bowel disease is induced by repeated administration of DSS at regular intervals in comparison with those by prescription drugs, tofacitinib, 5-ASA (5-acetyl salicylic acid), and prednisolone used in the clinic, FIG. 11A illustrates the body weight change, and FIG. 11B illustrates the mortality.

### [Description of Embodiments]

Hereinafter, the present invention will be described in more detail.

The present invention provides a pharmaceutical composition for use in the prevention or treatment of inflammatory bowel disease or ischemic colitis, which contains taurodeoxycholic acid or a pharmaceutically acceptable salt thereof as an active ingredient.

In the present invention, as the taurodeoxycholic acid, those isolated from animal carcasses, for example, animals such as sheep, dogs, goats, or rabbits, commercially available ones, and synthesized ones may be all safely used.

The taurodeoxycholic acid is a kind of bile acid, is in the form of taurine-conjugated deoxycholic acid, and has a chemical structure represented by the following [Chemical Formula 1], more specifically a chemical structure represented by the following [Chemical Formula 2] .

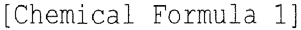

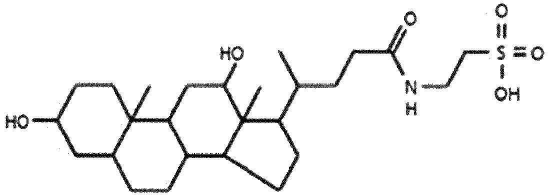

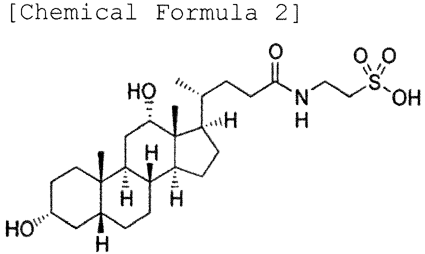

In the present invention, the taurodeoxycholic acid or a pharmaceutically acceptable salt thereof can inhibit the production of pro-inflammatory cytokines, more specifically the production of IL-6, IL-1β, or TNF-α.

The taurodeoxycholic acid or a pharmaceutically acceptable salt thereof can exhibit an equivalent or higher efficacy as compared with tofacitinib, 5-ASA (5-acetyl salicylic acid), and steroid drugs that are all currently used in the clinic for the treatment of ulcerative colitis.

The taurodeoxycholic acid or a pharmaceutically acceptable salt thereof can decrease the number of inflammatory cells in the bowels.

In the present invention, the inflammatory bowel disease may be ulcerative colitis, collagenous colitis, lymphoid colitis, diversion colitis, Crohn's disease, Behcet's syndrome or indeterminate colitis.

In a specific embodiment of the present invention, the present inventors administered sodium taurodeoxycholic acid to a mouse model in which inflammatory bowel disease was induced by administration of dextran sodium sulfate (DSS) before or after the DSS administration, analyzed clinical symptoms and histopathological symptoms, and as a result, confirmed that the clinical symptoms appearing in inflammatory bowel disease-induced mouse model such as body weight loss, diarrhea, and bloody stool and the histopathological symptoms such as a decrease in colon length, infiltration of inflammatory cells into mucous membrane, damage to the intestinal crypt, and ulcer were alleviated by the administration of sodium taurodeoxycholic acid (see FIGS. 1, 2, and 6). The clinical symptoms (body weight change and DAI change) were evaluated in comparison with those by clinical prescription drugs, tofacitinib and 5-ASA, and as a result, TDCA was confirmed to exhibit an equivalent or higher effect in an administration dose of 2.5 mg/kg, namely, a dose lower than the dose of the clinical prescription drugs (see FIG. 9).

The present inventors administered sodium taurodeoxycholic acid to a mouse model in which inflammatory bowel disease was induced by administration of DSS before or after the DSS administration, analyzed the inflammatory cells and pro-inflammatory cytokines, and as a result, confirmed that the increase in inflammatory cells and pro-inflammatory cytokines was suppressed in the intestinal tissue of the inflammatory bowel disease-induced mouse model by the administration of sodium taurodeoxycholic acid. An equivalent effect was confirmed in IL-6 and TNF-α inhibition and an equivalent or higher effect was confirmed in IL-1β inhibition as compared with those by the clinical prescription drugs, tofacitinib and 5-ASA (see FIGS. 3, 4, 7, and 9).

The present inventors administered sodium taurodeoxycholic acid at different concentrations to a mouse model in which inflammatory bowel disease was induced by administration of DSS, analyzed clinical symptoms and histopathological symptoms, and as a result, confirmed that superior effects were exhibited when sodium taurodeoxycholic acid was administered at an administration concentration of 2.5 to 10 mg/kg (see FIGS. 5 and 6). This result may be interpreted similarly to that the TDCA-administered group exhibits an equivalent or higher effect when the clinical symptoms (body weight change and mortality) in a mouse model in which chronic inflammatory bowel disease was induced by repeated administration of DSS in the case of TDCA 2.5 mg/kg BID (administered two times a day) or TDCA 2.5 mg/kg TID (administered 3 times a day) were compared with those in the case of clinical prescription drugs (see FIG. 11).

In addition, the present inventors confirmed that the excellent effect of TDCA on ulcerative colitis was due to the inhibition of pro-inflammatory cytokine secretion of macrophages that are humoral immune cells by TDCA (see FIG. 10).

Hence, the present inventors have confirmed that the clinical symptoms and histopathological symptoms caused by inflammatory bowel disease are alleviated and the increase in inflammatory cells in bowels and the production of pro-inflammatory cytokines are suppressed by using a salt of taurodeoxycholic acid, and thus taurodeoxycholic acid and a pharmaceutically acceptable salt thereof can be used as an active ingredient of a pharmaceutical composition for prevention or treatment of inflammatory bowel disease.

The present invention includes not only taurodeoxycholic acid represented by Chemical Formula 1 but also pharmaceutically acceptable salts thereof and all possible solvates, hydrates, racemates, or stereoisomers which may be prepared therefrom.

Taurodeoxycholic acid represented by Chemical Formula 1 of the present invention may be used in the form of a pharmaceutically acceptable salt, and an acid addition salt formed from a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid, or phosphorous acid and non-toxic organic acids such as aliphatic mono- and dicarboxylates, phenyl-substituted alkanoates, hydroxy alkanoates and alkandioates, aromatic acids, and aliphatic and aromatic sulfonic acids. Such pharmaceutically non-toxic salts include sulfates, pyrosulfates, bisulfates, sulfites, bisulfites, nitrates, phosphates, monohydrogen phosphates, dihydrogen phosphates, metaphosphates, pyrophosphates, chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butine-1,4-dioates, hexane-1,6-dioates, benzoates, chlorobenzoates, methylbenzoates, dinitro benzoates, hydroxybenzoates, methoxybenzoates, phthalates, terephthalates, benzenesulfonates, toluenesulfonates, chlorobenzenesulfonates, xylenesulfonates, phenylacetates, phenylpropionates, phenylbutyrates, citrates, lactates, hydroxybutyrates, glycolates, malates, tartrates, methanesulfonates, propanesulfonates, naphthalene-1-sulfonates, naphthalene-2-sulfonates, or mandelates.

The acid addition salt according to the present invention may be prepared by a conventional method, for example, by dissolving taurodeoxycholic acid represented by Chemical Formula 1 in an excessive amount of acid aqueous solution and precipitating this salt using a water-miscible organic solvent, for example, methanol, ethanol, acetone, or acetonitrile. The acid addition salt may also be prepared by evaporating the solvent or the excess acid from this mixture and drying the residue or by subjecting the precipitated salt to suction filtration.

Pharmaceutically acceptable metal salts may be prepared using bases. Alkali metal or alkaline earth metal salts are obtained, for example, by dissolving a compound in an excessive amount of alkali metal hydroxide or alkaline earth metal hydroxide solution, filtering the undissolved compound salt, and evaporating and drying the filtrate. At this time, it is pharmaceutically suitable to prepare a sodium, potassium, or calcium salt, more specifically it is suitable to prepare a sodium salt as the metal salt. A silver salt corresponding to this is obtained by reacting an alkali metal or alkaline earth metal salt with a suitable silver salt (for example, silver nitrate).

When the composition is formulated, the composition is prepared using diluents or excipients such as fillers, extenders, binders, wetting agents, disintegrants, and surfactants which are usually used.

The composition may be administered orally or parenterally and may be administered by transdermal administration, intraperitoneal administration, intravenous administration, intramuscular administration, subcutaneous administration, intradermal administration, or topical administration when being administered parenterally.

Solid preparations for oral administration include tablets, pills, powders, granules, capsules, troches and the like, and such solid preparations are prepared by mixing at least one or more excipients, for example, starch, calcium carbonate, sucrose or lactose, or gelatin with one or more taurodeoxycholic acids represented by Chemical Formula 1 of the present invention. In addition to simple excipients, lubricants such as magnesium stearate and talc are also used. Liquid preparations for oral administration include suspensions, oral liquids, emulsions, or syrups and may contain various excipients such as wetting agents, sweetening agents, fragrances, and preservatives in addition to water and liquid paraffin which are commonly used simple diluents.

Formulations for parenteral administration include sterilized aqueous solutions, non-aqueous solvents, suspension solvents, emulsions, freeze-dried preparations, suppositories, and the like.

As the non-aqueous solvent and the suspension solvent, propylene glycol, polyethylene glycol, vegetable oil such as olive oil, injectable esters such as ethyl oleate, and the like may be used. As the base for suppositories, witepsol, macrogol, tween 61, cacao butter, laurin, glycerol, gelatin and the like may be used.

The composition according to the present invention is administered in a pharmaceutically effective amount. In the present invention, the "pharmaceutically effective amount" means an amount sufficient to treat a disease at a reasonable benefit/risk ratio applicable to medical treatment. The effective dose level may be determined depending on the factors including the type and severity of patient's disease, the activity of the drug, the sensitivity to drugs, the time of administration, the route of administration and rate of excretion, the treatment period, and the drugs used concurrently, and other factors well known in the medical field. The composition of the present invention may be administered as an individual therapeutic agent or in combination with other therapeutic agents, may be administered sequentially or simultaneously with a conventional therapeutic agent, and may be administered singly or multiply. It is important to take into account all of the above factors and administer the composition in an amount so that the maximum effect can be obtained in the minimum amount without side effects, and the amount may be readily determined by those skilled in the art.

Specifically, the effective amount of the compound according to the present invention may vary depending on the age, sex, and body weight of the patient. The compound may be administered daily or every other day in an amount of generally 0.1 mg to 100 mg, specifically 0.1 mg to 50 mg, more specifically 1 mg to 15 mg, even more specifically 2.5 mg to 10 mg per 1 kg of body weight, or the compound may be administered one to three times a day in a total amount of generally 0.1 mg to 100 mg, specifically 0.1 mg to 50 mg, more specifically 1 mg to 15 mg, even more specifically 2.5 mg to 10 mg per 1 kg of body weight. However, the dose may be increased or decreased depending on the route of administration, severity, sex, body weight, age and the like and does not thus limit the scope of the present invention in any way.

The present invention also provides a health functional food for use in the prevention or treatment of inflammatory bowel disease or ischemic colitis, which contains taurodeoxycholic acid or a pharmaceutically acceptable salt thereof as an active ingredient.

In the present invention, as the taurodeoxycholic acid, those isolated from animal carcasses, for example, animals such as sheep, dogs, goats, or rabbits, commercially available ones, and synthesized ones may be all safely used.

The taurodeoxycholic acid is a kind of bile acid, is in the form of taurine-conjugated deoxycholic acid, and has a chemical structure represented by [Chemical Formula 1] .

In the present invention, the taurodeoxycholic acid or a pharmaceutically acceptable salt thereof can inhibit the production of pro-inflammatory cytokines, more specifically the production of IL-6, IL-1β, or TNF-α.

The taurodeoxycholic acid or a pharmaceutically acceptable salt thereof can exhibit an equivalent or higher efficacy as compared with tofacitinib, 5-ASA (5-acetyl salicylic acid), and steroid drugs that are currently used in the clinic for the treatment of ulcerative colitis.

The taurodeoxycholic acid or a pharmaceutically acceptable salt thereof can decrease the number of inflammatory cells in the bowels.

In the present invention, the inflammatory bowel disease may be ulcerative colitis, collagenous colitis, lymphoid colitis, ischemic colitis, diversion colitis, Crohn's disease, Behcet's syndrome or indeterminate colitis.

The present inventors have confirmed that the clinical symptoms and histopathological symptoms caused by inflammatory bowel disease are alleviated and the increase in inflammatory cells in bowels and the production of pro-inflammatory cytokines are suppressed by using a salt of taurodeoxycholic acid, and thus taurodeoxycholic acid and a pharmaceutically acceptable salt thereof can be used as an active ingredient of a health functional food for prevention or improvement of inflammatory bowel disease.

There is no particular limitation on the kind of food to which taurodeoxycholic acid of the present invention is added. Examples of food to which the substances may be added include drinks, meat, sausages, bread, biscuits, rice cakes, chocolates, candies, snacks, confectionery, pizza, ramen, other noodles, gum, dairy products including ice cream, various soups, beverages, alcoholic beverages, vitamin complexes, dairy products, and processed dairy products, and the food includes all health functional foods in the usual sense.

Taurodeoxycholic acid of the present invention may be added to food as it is or used with other foods or food ingredients, and may be suitably used according to a conventional method. The mixing amount of the active ingredient may be appropriately determined depending on the purpose of use (for prevention or improvement). In general, the amount of the compound in the health functional food may be 0.1 to 90 parts by weight with respect to the total food weight. However, in the case of intaking the active ingredient for a long period of time for the purpose of health and hygiene or for the purpose of health control, the amount may be equal to or less than the above range but the active ingredient may be used in an amount equal to or more than the above range since there is no problem in terms of safety.

When the health food composition according to the present invention is a beverage composition, there is no particular limitation on other ingredients except that the compound as an essential ingredient is contained at the indicated proportion, and the beverage composition may contain various flavoring agents or natural carbohydrates as additional ingredients like ordinary beverages. Examples of the natural carbohydrates include conventional sugars such as monosaccharides, for example, glucose and fructose; disaccharides, for example, maltose and sucrose; and polysaccharides, for example, dextrin and cyclodextrin and sugar alcohols such as xylitol, sorbitol, and erythritol. As flavoring agents other than those described above, natural flavoring agents (taumatin, stevia extract (for example, rebaudioside A, glycyrrhizin, and the like) and synthetic flavoring agents (saccharin, aspartame, and the like) may be used advantageously. The proportion of the natural carbohydrates is generally about 1 to 20 g, preferably about 5 to 10 g per 100 of the composition of the present invention.

The health food composition according to the present invention may contain various nutrients, vitamins, minerals (electrolytes), flavoring agents such as synthetic flavoring agents and natural flavoring agents, coloring agents and thickening agents (cheese, chocolate, and the like), pectic acid and salts thereof, alginic acid and salts thereof, organic acids, protective colloidal thickeners, pH adjusters, stabilizers, preservatives, glycerin, alcohols, carbonation agents used in carbonated beverages, and the like. In addition, the health food composition may contain natural fruit juice and flesh for the manufacture of fruit juice beverages and vegetable beverages.

These ingredients may be used independently or in combination. The proportion of these additives is not limited but is generally selected in a range of 0.1 to about 20 parts by weight per 100 parts by weight of taurodeoxycholic acid of the present invention.

In the present invention, it has been confirmed that the clinical symptoms and histopathological symptoms caused by inflammatory bowel disease are alleviated and the increase in inflammatory cells in bowels and the production of pro-inflammatory cytokines are suppressed by using a salt of taurodeoxycholic acid, and thus taurodeoxycholic acid and a pharmaceutically acceptable salt thereof can be used as an active ingredient for prevention, improvement, or treatment of inflammatory bowel disease.

### [Examples]

Hereinafter, the present invention will be described in detail with reference to Examples, Experimental Examples, and Preparation Examples.

However, the following Examples, Experimental Examples, and Preparation Examples are merely illustrative of the present invention, and the contents of the present invention are not limited to the following Examples, Experimental Examples, and Preparation Examples.

### <Example 1> Preparation of inflammatory bowel disease-induced mouse model

An inflammatory bowel disease-induced mouse model using dextran sodium sulfate (DSS) was prepared by the following method.

Specifically, male C57BL/6 mice (7 to 12 weeks old, body weight of 18 to 30 g) were adapted for one week and used in the experiment. Animal rearing was carried out under the conditions of a temperature of 25°C and a day and night cycle (12 hours night/12 hours daytime), and feed and drinking water were freely fed. All the animals were managed according to the guidelines for use of experimental animals of Seoul National University IACUC (IACUC: SNU-151223-4-1). On the day of administration of DSS (MP Biomedicals, Santa Ana, CA, USA) (Day 0), mice were weighed and marked on the tail.

First, in order to optimize the concentration of DSS as a substance inducing inflammatory bowel disease, 2% and 5% DSS solutions as an inflammatory bowel disease inducing substance were administrated to male C57BL/6 mice for 7 to 10 days and 4 days, respectively, and then the onset of colitis was monitored by measuring the body weight, stool consistency, and bloody stool. Through monitoring, a 2% DSS solution was used in the experiment.

Next, male C57BL/6 mice were fed with a 2% DSS solution together with drinking water from Day 0 of the experiment to induce inflammatory bowel disease. DPBS or 10, 5, 2.5, or 1 mg/kg sodium taurodeoxycholic acid (TDCA) was administered in 0.1 ml by oral gavage one time a day from Day 1 of the experiment. Alternatively, 0.1 ml of 5 mg/kg TDCA was administered one time a day by oral gavage 2 days before DSS administration.

The 2% DSS solution was used after being prepared by dissolving DSS powder in sterilized distilled water and filtering the solution. The TDCA was dissolved in DPBS (Dulbecco's phosphate-buffered saline) and used.

In order to evaluate the clinical symptoms-improving efficacy in comparison with those by clinical prescription drugs, inflammatory bowel disease was induced by 7-day or repeated administration of 3% or 2.5% DSS solution, and tofacitinib 10 mg/kg, 5-ASA 100 mg/kg or 200 mg/kg, prednisolone 1 mg/kg, and TDCA 2.5 mg/kg were administered one time a day from the start date of DSS administration. From the start date of DSS administration, TDCA 2.5 mg/kg was administered one time a day (QD), two times a day (BID), and three times a day (TID) .

The disease activity indexes (DAI) including body weight, body weight loss, stool consistency, and bloody stool were measured daily. DAI was scored according to the following [Table 1].

**[Table 1]**

| Loss of body weight | | Stool consistency | | Bleeding | |
|---|---|---|---|---|---|
| None | 0 | Normal stool | 0 | Normal | 0 |
| 1%-5% | 1 | (easy to pick up and shape should not be greatly distorted in the process) | | 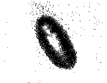 | |
| 5%-10% | 2 | Loose stool | 2 | Bloody stool | 2 |
| 10%-20% | 3 | (not easy to pick up and shape will be distorted easily) | | 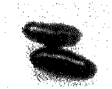 | |
| >20% | 4 | Diarrhea | 4 | Gross blood | 4 |
| | | (watery, no shape) | | 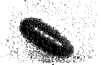 | |

### <Example 2> Analysis of histopathological symptom in inflammatory bowel disease-induced mouse model

After the experiment was completed, the mice were euthanized with CO₂ and the bowels were excised. Next, the bowels were put in cold 1 × DPBS and the remaining mesenteric adipose tissue was isolated. At this time, the colon length was measured. Next, the tail end of the colon was opened and wound up with scissors to obtain colon tissue. The obtained colon tissue was fixed in 10% neutral buffered formalin (Sigma-Aldrich, HT501128-4L, St. Louis, MO, USA) at room temperature for 48 hours and then treated in a tissue processor (Excelsior ES, Thermo scientific, Waltham, MA, USA) for 12 hours. The processed tissue was sectioned and paraffinized using a microtome (Microm, HM 340E, Thermo Scientific, Waltham, MA, USA) and an embedding system (HistoStar, Thermo Scientific, Waltham, MA, USA). Next, the processed tissue was stained with hematoxylin and eosin, and visualized under an optical microscope (Motic, BA310, Redding, CA, USA) to confirm the histopathological state, and the histopathological state was scored to histological score according to the criteria in the following [Table 2].

**[Table 2]**

| | |
|---|---|
| Severity of inflammation | • 0 - rare inflammatory cells in the lamina propria; |
| | • 1 - increased numbers of granulocytes in the lamina propria |
| | • 2 - confluence of inflammatory cells extending into the submucosa |
| | • 3 - transmural extension of the inflammatory infiltrate |
| Crypt damage | • 0 - intact crypts; |
| | • 1 - loss of the basal one-third |
| | • 2 - loss of the basal two-thirds |
| | • 3 - entire crypt loss |
| | • 4 - change of epithelial surface with erosion |
| | • 5 - confluent erosion |
| Ulceration | • 0 - absence of ulcer |
| | • 1 - 1 or 2 foci of ulcerations |
| | • 2 - 3 or 4 foci of ulcerations |
| | • 3 - confluent or extensive ulcerations |
| Crypt abscess | • 0 - absent |
| | • 1 - present |

### <Example 3> Analysis of inflammatory cell and pro-inflammatory cytokine in inflammatory bowel disease-induced mouse model

### <3-1> Analysis of pro-inflammatory cytokine

A colon was obtained from a mouse which had been subjected to an experiment by the same method as that described in <Example 2>, the peripheral colon was cut into 2 cm in length and the weight was recorded. Next, the colon was chopped into 1 mm pieces and frozen on ice, treated with 500 µl of cold DPBS containing protease inhibitor (Roche, 11836170001, Basel, Switzerland), and homogenized for 10 to 20 seconds using an electric homogenizer (IKA T10 Basic, Wilmington, SE, USA). The homogenate was centrifuged at 4°C for 10 minutes at 12,000 X g to obtain a supernatant. ELISA analysis was performed using the obtained supernatant, IL-6 DuoSet ELISA kit (R & D systems, USA), IL-1β DuoSet ELISA kit (R & D systems, USA), and TNF-α DuoSet ELISA kit (R & D systems, USA) according to the manufacturer's procedure to confirm the production of pro-inflammatory cytokines.

### <3-2> Analysis of inflammatory cell

A colon tissue was obtained from a mouse which had been subjected to an experiment by the same method as that described in <Example 2> and put into 30 ml of EDTA and dissociated by being stirred at 37°C for 30 minutes in order to remove mucus. Next, the tissue was drained with a strainer (70 um) and washed with warm DPBS. The washing process was repeated a total of 4 to 5 times. An enzyme mixture was prepared by adding 2.35 ml of RPMI 1640 or DMEM, 100 µl of enzyme D, 50 ul of enzyme R, and 12.5 µl of enzyme A to a gentleMACS C tube (Miltenyi Biotec, 130-096-334, Bergisch Gladbach, Germany). The tissue was transferred to the gentleMACS C tube containing the enzyme mixture and dissociated using the gentleMACS Dissociator (Miltenyi Biotec, 130-093-235, Bergisch Gladbach, Germany). Next, the reaction was conducted at 37°C for 40 minutes in a thermal incubator (Thermo scientific, BB-15, Waltham, MA, USA) under continuous rotation using a MACSmix Tube Rotator. The digested cells were resuspended in 4 ml of 40% Percoll solution. To 40% Percoll solution, 4 ml of 75% Percoll solution was added, and centrifugation was conducted at 25°C and 1200 rpm for 20 minutes. After centrifugation, the intermediate layer was recovered and resuspended in 15 ml of complete medium. The resuspended intermediate layer was centrifuged at 4°C and 1200 rpm for 7 minutes to obtain a pellet, and the pellet was resuspended in 3 ml of complete medium to obtain lamina propria mononuclear cells (LPMC).

Next, trypan blue exclusion analysis was performed in order to confirm the ratio of viable cells to dead cells. Specifically, 20 µl of the obtained LPMC was stained with 20 ul of a 0.4% trypan blue solution. Next, the viable cells were counted to calculate the cell viability. The cell viability was calculated by dividing the number of viable cells by the total number of cells.

In order to confirm changes in the number of inflammatory cells, FACS analysis was performed. Specifically, the obtained LPMC cells were treated with 0.5 µl of an anti-mouse CD16/CD32 purified monoclonal antibody per 1 × 10⁶ cells at room temperature for 10 minutes in order to block non-specific Fc-mediated interaction. Next, the cells were washed with FACS buffer (1 mM EDTA in 1% FBS and DPBS) and centrifuged at 4°C and 1200 rpm for 5 minutes. 1 × 10⁶ cells were divided into each FACS tube. Thereto, 0.5 µl of each primary antibody in 50 µl of FACS buffer was added, and reaction was conducted at room temperature for 10 minutes. Anti-CD3, anti-CD4, anti-CD8, anti-CD19, anti-CD11b, anti-CD11c, anti-CD45, anti-Ly6c, anti-Ly6g, and anti-MHCII antibodies were used as the primary antibodies. Next, the cells were washed two times with DPBS, resuspended in 200 µl DAPI, then analyzed using a flow cytometer (LSR Fortessa cytometer, Mississauga, Canada), and graphed.

### <3-3> Inhibition test on ability to secrete pro-inflammatory cytokine of bone marrow-derived macrophage in inflammatory bowel disease-induced mouse model

Bone marrow was collected from a DPBS-administered mouse which had been subjected to an experiment by the same method as that described in <Example 2>, induced to differentiate into macrophages, then pretreated with LPS (10 ng/ml) for 3 hours so as to be 4 × 10⁴ cells in a 96-well plate, treated with TDCA at various concentrations for 1 hour, then treated with ATP (0.5 mM) or BzATP (0.3 mM) for an additional 1 hour, and cultured, and the amount of IL-1β in the cell culture was measured and analyzed using an ELISA kit (R & D systems, USA) according to the manufacturer's procedure.

### <Example 4> Statistical analysis

Experimental results were expressed as the average ± SD (standard deviation) of three repeated experiments. Statistical significance was confirmed by one-way analysis of variance (ANOVA) with Tukey's HSD test. It was considered to be statistically significant when p < 0.05.

### <Experimental Example 1> Confirmation of clinical symptom alleviation by TDCA in inflammatory bowel disease-induced mouse model

In order to examine the therapeutic effect of sodium taurodeoxycholic acid (TDCA) on inflammatory bowel disease, clinical symptom analysis was performed after TDCA was administered to an inflammatory bowel disease-induced mouse model.

Specifically, female C57BL/6 mice were fed with a 2% DSS solution or DPBS together with drinking water for 10 days from Day 0 of the experiment by the same method as that described in <Example 1>, DPBS or 5 mg/kg TDCA was administered to the mice by oral gavage one time a day for 11 days from Day 1 of the experiment, and the body weight and DAI were measured daily. As a control, mice freely fed with drinking water were used.

As a result, as illustrated in FIG. 1, in the case of body weight change, it was confirmed that the body weight of the DSS and DPBS administration group (DSS+DPBS group) significantly decreased after 6 to 7 days of DSS administration and about 15% of the initial body weight was lost on Day 10. On the other hand, it was confirmed that the DSS and TDCA administration group (DSS+TDCA group) started to lose the body weight about 2 days later than the DSS+DPBS group and about 5% of the initial body weight was lost on Day 10. It was confirmed that the body weight of mice was gradually restored after the DSS administration was stopped (FIG. 1A).

In the case of DAI change, it was confirmed that DAI was high and the inflammation was severe in the DSS+DPBS group. In particular, it was confirmed that 60% (4 out of 6) of mice developed diarrhea and severe bloody stool after 3 to 5 days of DSS administration. On the other hand, it was confirmed that the DAI in the DSS+TDCA group was lower than that in the DSS+DPBS group and the symptoms were alleviated in the DSS+TDCA group (FIG. 1B).

In the case of the survival rate of mice, it was confirmed that the survival rate was 60% in the DSS+DPBS group but the survival rate was 100% in the DSS+TDCA group (FIG. 1C).

As illustrated in FIG. 9, the clinical symptoms and mortality were evaluated in comparison with those by clinical prescription drugs, and as a result, it was confirmed that TDCA 2.5 mg/kg exhibited an effect equivalent to those by tofacitinib 10 mg/kg and 5-ASA 100 mg/kg on inflammatory bowel disease induced by 3% DSS administration for 7 days in terms of the body weight change and DAI change (FIGS. 9A and 9B).

As illustrated in FIG. 11, in the chronic inflammatory bowel disease model induced with 2.5% DSS, it was confirmed that TDCA 2.5 mg/kg exhibited an effect equivalent to or higher than those by tofacitinib 10 mg/kg and 5-ASA 200 mg/kg or the body weight change in all the administration methods of QD, BID, and TID and exhibited an effect equivalent to or higher than those by clinical prescription drugs on the mortality as well (FIGS. 11A and 11B).

From the above results, it has been confirmed that TDCA alleviates clinical symptoms such as body weight loss, diarrhea, and bloody stool caused by inflammatory bowel disease.

### <Experimental Example 2> Confirmation of histopathological symptom alleviation by TDCA in inflammatory bowel disease-induced mouse model

In inflammatory bowel disease, ulcer formation, mucosal edema, goblet cell loss, crypt distortion, and abscesse are caused by the infiltration of inflammatory cells into intestinal mucosa and lamina propria. Hence, in order to examine the therapeutic effect of TDCA on inflammatory bowel disease, histopathological symptom analysis was performed after TDCA was administered to an inflammatory bowel disease-induced mouse model.

Specifically, female C57BL/6 mice were fed with a 2% DSS solution or DPBS together with drinking water for 7 days from Day 0 of the experiment by the same method as that described in <Example 1>, DPBS or 5 mg/kg TDCA was administered to the mice by oral gavage one time a day for 6 days from Day 1 of the experiment, and the body weight and DAI were measured daily. As a control, mice freely fed with drinking water were used. Next, the mice were sacrificed on Days 5 and 7 of the experiment by the same method as that described in <Example 2>, and histopathological symptom analysis was performed.

As a result, as illustrated in FIG. 2, it was confirmed that the colon length remarkably decreased in the DSS and DPBS administration group (DDS+DPBS group) but the decrease in colon length was slight in the DSS and TDCA administration group (DSS+TDCA group) (FIG. 2A). It was confirmed that infiltration of inflammatory cells into lamina propria, damage to the entire intestinal crypt, and changes in the epithelial surface due to infiltration were observed in the DDS+DPBS group, but in the DSS+TDCA group, epithelial cell defects were not observed similarly to the control group and the infiltration of inflammatory cells into the mucous membrane was slight (FIG. 2B). It was confirmed that the average histological score was as high as about 7 and the ulcer was severe in the DDS+DPBS group, but the average histological score was about 4 and the ulcer was alleviated in the DSS+TDCA group (FIG. 2C).

As illustrated in FIG. 9, the colon length and histological lesion index were evaluated in comparison with those by clinical prescription drugs, and as a result, it was confirmed that TDCA 2.5 mg/kg exhibited an effect equivalent to those by tofacitinib 10 mg/kg and 5-ASA 100 mg/kg on inflammatory bowel disease induced by 3% DSS administration for 7 days in terms of the colon length and histological lesion index (FIGS. 9C, 9D, and 9E) .

From the above results, it has been confirmed that TDCA alleviates histopathological symptoms such as infiltration of inflammatory cells, damage to colon mucosal epithelial cells, and damage to intestinal crypt caused by inflammatory bowel disease.

### <Experimental Example 3> Confirmation of decrease in inflammatory cell and pro-inflammatory cytokine by TDCA in inflammatory bowel disease-induced mouse model

In inflammatory bowel disease, the production of pro-inflammatory cytokines such as IL-1, IL-6, TNF-α, and chemokines increases along with the infiltration of inflammatory cells into intestinal mucosa and lamina propria and the production of anti-inflammatory cytokines such as IL-10 is downregulated. Hence, in order to examine the therapeutic effect of TDCA on inflammatory bowel disease, inflammatory cell and pro-inflammatory cytokine analysis was performed after TDCA was administered to an inflammatory bowel disease-induced mouse model.

Specifically, female C57BL/6 mice were fed with a 2% DSS solution together with drinking water for 7 days from Day 0 of the experiment by the same method as that described in <Example 1>, DPBS or 5 mg/kg TDCA was administered to the mice by oral gavage one time a day for 6 days from Day 1 of the experiment, and the body weight and DAI were measured daily. As a control, mice freely fed with drinking water were used. Next, the mice were sacrificed on Day 7 of the experiment by the same method as that described in <Example 3>, and inflammatory cell and proinflammatory cytokine analysis was performed.

As a result, as illustrated in FIG. 3, it was confirmed that the numbers of CD11b⁺, Ly6g⁺, and Ly6c⁺ LPMCs in the colon tissue increased in the DSS+DPBS group but decreased in the DSS+TDCA group (Fig. 3A). It was confirmed that IL-6 and IL-1β in the colon tissue increased in the DSS+DPBS group but decreased in the DSS+TDCA group (FIG. 3B).

As illustrated in FIG. 9, the concentrations of IL-6, IL-1β and TNF-α in the colon tissue were evaluated in comparison with those by clinical prescription drugs, and as a result, it was confirmed that TDCA 2.5mg/kg more effectively suppressed the concentration of IL-1β than tofacitinib 10 mg/kg and 5-ASA 100 mg/kg, and TDCA 2.5mg/kg suppressed the concentrations of IL-6 and TNF-α to the extent equivalent to those by tofacitinib 10 mg/kg and 5-ASA 100 mg/kg in inflammatory bowel disease induced by 3% DSS administration for 7 days (Fig. 9F).

It has been confirmed that the reason why TDCA exhibits superior effect to the control drugs in suppression of the concentration of pro-inflammatory cytokine IL-1β in this way is because TDCA inhibits the inflammasome activity of bone marrow-derived macrophages at IC₅₀ = 60 to 90 nM (FIGS. 10A and 10B).

From the above results, it has been confirmed that TDCA suppresses the increase in inflammatory cells and pro-inflammatory cytokines in the bowels by inflammatory bowel disease.

### <Experimental Example 4> Confirmation of clinical symptom and histopathological symptom alleviation at various TDCA concentrations in inflammatory bowel disease-induced mouse model

### <4-1> Confirmation of clinical symptom alleviation at various TDCA concentrations in inflammatory bowel disease-induced mouse model

In order to examine the therapeutic effect on inflammatory bowel disease depending on the TDCA concentration, clinical symptom analysis was performed after TDCA was administered to an inflammatory bowel disease-induced mouse model at various concentrations.

Specifically, female C57BL/6 mice were fed with a 2% DSS solution or DPBS together with drinking water for 10 days from Day 0 of the experiment by the same method as that described in <Example 1>, DPBS or 10, 5, 2.5, or 1 mg/kg TDCA was administered to the mice by oral gavage one time a day for 11 days from Day 1 of the experiment, and the body weight and DAI were measured daily. As a control, mice freely fed with drinking water were used.

As a result, as illustrated in FIG. 4, it was confirmed that the body weight of the DSS and DPBS administration group (DSS+DPBS group) significantly decreased after 6 to 7 days of DSS administration but the DSS and TDCA administration group (DSS+TDCA group) started to lose the body weight about 2 to 3 days later than the DSS+DPBS group and the degree of alleviation of body weight loss was superior when TDCA was administered at concentrations of 5 and 10 mg/kg. It was confirmed that the body weight of mice was gradually restored after the DSS administration was stopped (FIG. 4A). In the case of DAI change, it was confirmed that DAI was remarkably high and the inflammation was severe in the DSS+DPBS group. On the other hand, it was confirmed that the DAI in the DSS+TDCA group was lower than that in the DSS+DPBS group and DAI was lower when TDCA was administered at concentrations of 10, 5, and 2.5 mg/kg (FIG. 4B).

### <4-2> Confirmation of histopathological symptom alleviation at various TDCA concentrations in inflammatory bowel disease-induced mouse model

In order to examine the therapeutic effect on inflammatory bowel disease depending on the TDCA concentration, histopathological symptom analysis was performed after TDCA was administered to an inflammatory bowel disease-inducing mouse model at various concentrations.

Specifically, female C57BL/6 mice were fed with a 2% DSS solution or DPBS together with drinking water for 7 days from Day 0 of the experiment by the same method as that described in <Example 1>, DPBS or 10, 5, 2.5, or 1 mg/kg TDCA was administered to the mice by oral gavage one time a day for 6 days from Day 1 the experiment, and the body weight and DAI were measured daily. As a control, mice freely fed with drinking water were used.

Next, the mice were sacrificed on Day 8 of the experiment by the same method as that described in <Example 2>, and histopathological symptom analysis was performed.

As a result, as illustrated in FIG. 5, it was confirmed that the colon length remarkably decreased in the DSS and DPBS administration group (DDS+DPBS group). However, it was confirmed that the decrease in colon length was slight in the DSS and TDCA administration group (DSS+TDCA group), in particular the decrease in colon length was slighter when TDCA was administered at concentrations of 5 and 10 mg/kg (FIG. 5A). It was confirmed that infiltration of inflammatory cells into lamina propria, damage to the entire intestinal crypt, and changes in the epithelial surface due to infiltration were observed in the DDS+DPBS group but epithelial cell defects were not observed similarly to the control group and the infiltration of inflammatory cells into the mucous membrane was alleviated in the DSS+TDCA group. It was confirmed that the average histological score was about 4 and the ulcer alleviating effect was superior in particular when TDCA was administered at concentrations of 5 and 10 mg/kg (FIGS. 5B and 5C).

From the results of Experimental Examples <4-1> and <4-2>, it has been confirmed that TDCA concentrations of 2.5 to 10 mg/kg as a TDCA administration concentration are more effective on the alleviation of clinical symptoms and histopathological symptoms caused by inflammatory bowel disease.

### <Experimental Example 5> Confirmation of clinical symptom and histopathological symptom alleviation and decrease in inflammatory cell and pro-inflammatory cytokine by TDCA administration before or after induction of inflammatory bowel disease in inflammatory bowel disease-induced mouse model

### <5-1> Confirmation of clinical symptom alleviation by TDCA administration before or after induction of inflammatory bowel disease

In order to examine the preventive effect of TDCA on inflammatory bowel disease, clinical symptom analysis was performed after TDCA was administered to an inflammatory bowel disease-induced mouse model before or after induction of inflammatory bowel disease.

Specifically, female C57BL/6 mice were fed with a 2% DSS solution together with drinking water for 7 days from Day 0 of the experiment by the same method as that described in <Example 1>, DPBS or 5 mg/kg TDCA was administered to the mice by oral gavage from Day 1 of DSS administration or 2 days before DSS administration, and the body weight and DAI were measured daily. As a control, mice freely fed with drinking water were used.

The mice were sacrificed on Day 8 of the experiment by the same method as that described in <Example 2>, and the colon length was measured.

As a result, as illustrated in FIG. 6, it was confirmed that the body weight loss rate in the group (DSS+preTDCA group) administered with TDCA before induction of inflammatory bowel disease with DSS was similar to that in the group (DSS+TDCA group) administered with TDCA after induction of inflammatory bowel disease with DSS (FIG. 6A). It was confirmed that DAI similarly decreased in both the DSS+preTDCA group and the DSS+TDCA group (FIG. 6B). In addition, it was confirmed that the decrease in colon length was suppressed in both the DSS+preTDCA group and the DSS+TDCA group (FIG. 6C).

### <5-2> Confirmation of decrease in inflammatory cell and pro-inflammatory cytokine by TDCA administration before or after induction of inflammatory bowel disease

In order to examine the preventive effect of TDCA on inflammatory bowel disease, inflammatory cell and pro-inflammatory cytokine analysis was performed after TDCA was administered to an inflammatory bowel disease-induced mouse model before or after induction of inflammatory bowel disease.

Specifically, female C57BL/6 mice were fed with a 2% DSS solution together with drinking water for 7 days from Day 0 of the experiment by the same method as that described in <Example 1>, DPBS or 5 mg/kg TDCA was administered to the mice by oral gavage from Day 1 of DSS administration or 2 days before DSS administration, and the body weight and DAI were measured daily. As a control, mice freely fed with drinking water were used. Next, the mice were sacrificed on Day 7 of the experiment by the same method as that described in <Example 3>, and the inflammatory cell and proinflammatory cytokine analysis was performed.

As a result, as illustrated in FIG. 7, it was confirmed that the numbers of CD11b⁺, Ly6g⁺, Ly6c⁺, CD3⁺, CD4⁺, CD8⁺, CD19⁺, and MHCII⁺CD11c⁺LPMCs in the colon tissue increased in the DSS+DPBS group but decreased in both the DSS+preTDCA group and the DSS+TDCA group (FIG. 7A). It was confirmed that IL-6, IL-1β, and TNF-α in the colon tissue increased in the DSS+DPBS group but decreased in both the DSS+preTDCA group and the DSS+TDCA group (FIG. 7B).

From the results of Experimental Examples <5-1> and <5-2>, it has been confirmed that TDCA can not only treat inflammatory bowel disease but also prevent the onset of inflammatory bowel disease.

### <Experimental Example 6> Confirmation of clinical symptom change by TDCA administration in inflammatory bowel disease-induced TGR5^{-/-} mouse model

In order to find out the mechanism of treatment or prevention of inflammatory bowel disease by TDCA, inflammatory bowel disease was induced in a TGR5 knock-out mouse model, TDCA was administered to the mouse model, and then clinical symptom analysis was performed.

Specifically, C57BL/6-Gpbar1^{tm1(KOMP)Vlcg} mice (TGR5 KO, KOMP Repository, The Knockout Mouse Project, University of California, Davis, CA) were used as TGR5 knockout (TGR5^{-/-}) female C57BL/6 mice. The TGR5^{-/-} female C57BL/6 mice or TGR5 normal (WT) female C57BL/6 mice were fed with a 2% DSS solution or DPBS together with drinking water for 10 days from Day 0 of the experiment by the same method as that described in <Example 1>. DPBS or 5 mg/kg TDCA was administered to the mice by oral gavage one time a day for 11 days from Day 1 of the experiment, and the body weight and DAI were measured daily. As a control, mice freely fed with drinking water were used.

As a result, as illustrated in FIG. 8, in the case of body weight change, it was confirmed that the body weight of the TGR5^{-/-} group (DSS+TDCA (TGR5^{-/-}) group) administered with DSS and TDCA significantly decreased after 5 to 6 days of DSS administration and about 27% of the initial body weight was lost on Day 10. On the other hand, it was confirmed that the body weight of the TGR5 normal group (DSS+TDCA (WT) group) administered with DSS and TDCA started to decrease about 2 to 3 days later than the other groups and about 5% of the initial body weight was lost on Day 10. It was confirmed that the body weight of mice was gradually restored after the DSS administration was stopped (FIG. 8A).

In the case of DAI change, it was confirmed that DAI was high and the inflammation was severe in the DSS+TDCA (TGR5^{-/-}) group. On the other hand, it was confirmed that the DAI in the DSS+TDCA (WT) group was lower than those in the other groups and the symptoms were alleviated in the DSS+TDCA (WT) group (FIG. 8B).

In the case of the survival rate of mice, it was confirmed that the survival rate was 60% in the DSS+TDCA (TGR5^{-/-}) group but the survival rate was 100% in the DSS+TDCA (WT) group (FIG. 8C).

From the above results, it has been confirmed that TDCA alleviates clinical symptoms such as body weight loss, diarrhea, and bloody stool caused by inflammatory bowel disease through the mediation of TGR5.

Hereinafter, Preparation Examples of each formulation according to the present invention will be described. The following Preparation Examples are intended to aid understanding of the practice of the present invention, but it does not mean that the preparation method of the formulation according to the present invention is limited to the following Preparation Examples.

### <Preparation Example 1> Preparation of drug

### <1-1> Preparation of powder

Taurodeoxycholic acid 10 mg
Sucrose 100 mg
Talc 10 mg

The ingredients are powdered, mixed, and then filled in an airtight pouch to prepare a powder.

### <1-2> Preparation of tablet

| | |
|---|---|
| Taurodeoxycholic acid | 10 mg |
| Starch | 100 mg |
| Sucrose | 100 mg |
| Magnesium stearate | 2 mg |

The ingredients are mixed together by a conventional tablet preparing method and then tableted to prepare a tablet.

### <1-3> Preparation of capsule

| | |
|---|---|
| Taurodeoxycholic acid | 10 mg |
| Crystalline cellulose | 3 mg |
| Lactose | 15 mg |
| Magnesium stearate | 1 mg |

The ingredients are mixed together by a conventional capsule preparing method and then filled into a gelatin capsule to prepare a capsule.

### <1-4> Preparation of granule

| | |
|---|---|
| Taurodeoxycholic acid | 10 mg |
| Soybean extract | 50 mg |
| Glucose | 200 mg |
| Starch | 500 mg |

The ingredients are mixed together, then 100 mL of 30% ethanol is added to the mixture, this mixture is dried at 60°C to form granules, and then the granules are filled into a pouch to prepare granules.

### <1-5> Preparation of pill

| | |
|---|---|
| Taurodeoxycholic acid | 10 mg |
| Lactose | 1,500 mg |
| Glycerin | 1,500 mg |
| Starch | 980 mg |

The ingredients are mixed together and then pills are prepared so as to be 4 g per pill by a conventional pill preparing method.

### <1-6> Preparation of injection

| | |
|---|---|
| Taurodeoxycholic acid | 10 mg |
| Mannitol | 180 mg |
| Sterile distilled water for injection | 2,870 mg |
| Na₂HPO₄12H₂O | 30 mg |

An injection is prepared by mixing the ingredients together so as to be 2 mL per ampoule by a conventional injection preparing method.

### <1-7> Preparation of liquid formulation

| | |
|---|---|
| Taurodeoxycholic acid | 10 mg |
| Isomerized glucose syrup | 10,000 mg |
| Mannitol | 5,000 mg |
| Purified water | appropriate amount |

The ingredients are dissolved in purified water by a conventional liquid formulation preparing method, an appropriate fragrance is added to the solution, and then this solution is filled into a bottle and sterilized to prepare a liquid formulation.

### <Preparation Example 2> Preparation of food

### <2-1> Preparation of flour food

To flour, 0.5 to 5.0 parts by weight of taurodeoxycholic acid of the present invention was added, and bread, cakes, cookies, crackers, and noodles were prepared using this mixture.

### <2-2> Preparation of soup and gravy

Meat products for health promotion, noodle soups, and gravies were prepared by adding 0.1 to 5.0 parts by weight of taurodeoxycholic acid of the present invention to soups and gravies.

### <2-3> Preparation of ground beef

Ground beef for health promotion was prepared by adding 10 parts by weight of taurodeoxycholic acid of the present invention to ground beef.

### <2-4> Preparation of dairy product

To milk, 5 to 10 parts by weight of taurodeoxycholic acid of the present invention was added, and various dairy products such as butter and ice cream were prepared using the milk.

### <2-5> Preparation of Seonsik

Brown rice, barley, glutinous rice, and adlay were gelatinized and dried by a known method, and then roasted, and then prepared into a powder having a particle size of 60 mesh using a grinder.

Black soybean, black sesame seed, and perilla seed were also steamed and dried by a known method, and then roasted, and then prepared into a powder having a particle size of 60 mesh using a grinder.

Taurodeoxycholic acid of the present invention was concentrated under reduced pressure in a vacuum concentrator, sprayed, and dried using a hot air dryer, and the obtained dry product was ground using a grinder to a particle size of 60 mesh to obtain a dry powder.

Seonsik was prepared by mixing the grains, seeds, and taurodeoxycholic acid of the present invention prepared above together at the following proportions.
Grains (30 parts by weight of brown rice, 15 parts by weight of adlay, 20 parts by weight of barley),
Seeds (7 parts by weight of perilla seed, 8 parts by weight of black soybean, 7 parts by weight of black sesame seed),
Taurodeoxycholic acid of the present invention (3 parts by weight),
Ganoderma lucidum (0.5 parts by weight), and
Rehmannia root (0.5 parts by weight)

### <Preparation Example 3> Preparation of beverage

### <3-1> Preparation of health drink

Subsidiary materials such as liquid fructose (0.5%), oligosaccharide (2%), sugar (2%), salt (0.5%), and water (75%) and 5 g of taurodeoxycholic acid of the present invention were homogeneously mixed together and sterilized for a short time, and then packaged in a small container such as a glass bottle or a plastic bottle to prepare a health drink.

### <3-2> Preparation of vegetable juice

Vegetable juice was prepared by adding 5 g of taurodeoxycholic acid of the present invention to 1,000 ml of tomato or carrot juice.

### <3-3> Preparation of fruit juice

Fruit juice was prepared by adding 1 g of taurodeoxycholic acid of the present invention to 1,000 ml of apple or grape juice.

### [Industrial Applicability]

In the present invention, it has been confirmed that the clinical symptoms and histopathological symptoms caused by inflammatory bowel disease are alleviated and the increase in inflammatory cells in bowels and the production of pro-inflammatory cytokines are suppressed by using a salt of taurodeoxycholic acid, and thus taurodeoxycholic acid and a pharmaceutically acceptable salt thereof can be used as an active ingredient of a composition for prevention or treatment of inflammatory bowel disease.

## Claims

1. A pharmaceutical composition for use in the prevention or treatment of inflammatory bowel disease or ischemic colitis, the pharmaceutical composition comprising taurodeoxycholic acid or a pharmaceutically acceptable salt thereof as an active ingredient.

2. The pharmaceutical composition for use according to claim 1, wherein the taurodeoxycholic acid is a compound represented by the following [Chemical Formula 1]:

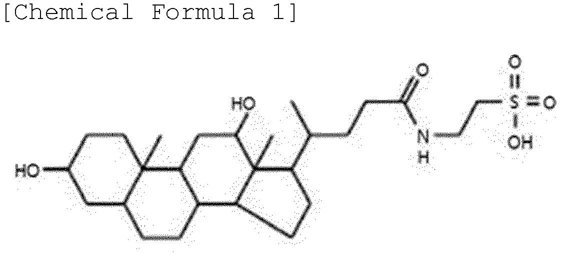

3. The pharmaceutical composition for use according to claim 1, wherein the pharmaceutically acceptable salt is a sodium salt.

4. The pharmaceutical composition for use according to claim 1, wherein the taurodeoxycholic acid or a pharmaceutically acceptable salt thereof inhibits production of pro-inflammatory cytokines.

5. The pharmaceutical composition for use according to claim 4, wherein the pro-inflammatory cytokines are any one or more selected from the group consisting of IL-6, IL-1β, and TNF-α.

6. The pharmaceutical composition for use according to claim 1, wherein the taurodeoxycholic acid or a pharmaceutically acceptable salt thereof decreases a number of inflammatory cells in bowels.

7. The pharmaceutical composition for use according to claim 1, wherein the inflammatory bowel disease is selected from ulcerative colitis, collagenous colitis, lymphoid colitis, diversion colitis, Crohn's disease, Behcet's syndrome, and indeterminate colitis.

8. A health food for use in the prevention or improvement of inflammatory bowel disease or ischemic colitis, the health food comprising taurodeoxycholic acid or a pharmaceutically acceptable salt thereof as an active ingredient.

9. The health food for use according to claim 8, wherein the inflammatory bowel disease is selected from ulcerative colitis, collagenous colitis, lymphoid colitis, diversion colitis, Crohn's disease, Behcet's syndrome, and indeterminate colitis.

## Patentansprüche

1. Pharmazeutische Zusammensetzung zur Verwendung bei der Vorbeuge oder Behandlung chronisch-entzündlicher Darmerkrankungen oder ischämischer Kolitis, wobei die pharmazeutische Zusammensetzung Taurodeoxycholsäure oder ein pharmazeutisch verträgliches Salz davon als Wirkstoff aufweist.

2. Pharmazeutische Zusammensetzung zur Verwendung nach Anspruch 1, wobei die Taurodeoxycholsäure eine Verbindung ist, die durch die folgende [Chemische Formel 1] dargestellt wird:

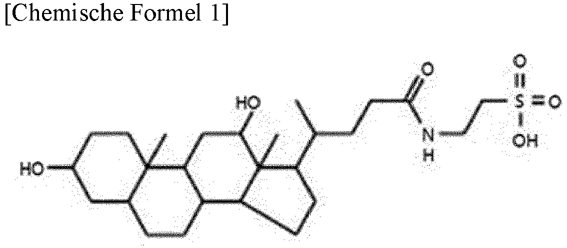

3. Pharmazeutische Zusammensetzung zur Verwendung nach Anspruch 1, wobei das pharmazeutisch verträgliche Salz ein Natriumsalz ist.

4. Pharmazeutische Zusammensetzung zur Verwendung nach Anspruch 1, wobei die Taurodeoxycholsäure oder das pharmazeutisch verträgliche Salz davon die Produktion von proinflammatorischen Zytokinen hemmt.

5. Pharmazeutische Zusammensetzung zur Verwendung nach Anspruch 4, wobei die proinflammatorischen Zytokine Zytokine eines oder mehrerer Typen sind, die aus der Gruppe ausgewählt sind, die IL-6, IL-1β und TNF-α umfasst.

6. Pharmazeutische Zusammensetzung zur Verwendung nach Anspruch 1, wobei die Taurodeoxycholsäure oder das pharmazeutisch verträgliche Salz davon die Anzahl inflammatorischer Zellen im Darm reduziert.

7. Pharmazeutische Zusammensetzung zur Verwendung nach Anspruch 1, wobei die chronisch-entzündliche Darmerkrankung aus Colitis ulcerosa, kollagener Kolitis, lymphoider Kolitis, Diversionskolitis, Morbus Crohn, Morbus Behcet und Colitis indeterminata ausgewählt ist.

8. Gesundheitskost zur Verwendung bei der Vorbeuge oder Besserung chronisch-entzündlicher Darmerkrankungen oder ischämischer Kolitis, wobei die Gesundheitskost Taurodeoxycholsäure oder ein pharmazeutisch verträgliches Salz davon als Wirkstoff aufweist.

9. Gesundheitskost zur Verwendung nach Anspruch 8, wobei die chronisch-entzündliche Darmerkrankung aus Colitis ulcerosa, kollagener Kolitis, lymphoider Kolitis, Diversionskolitis, Morbus Crohn, Morbus Behcet und Colitis indeterminata ausgewählt ist.

## Revendications

1. Composition pharmaceutique destinée à être utilisée pour la prévention ou le traitement d'une maladie inflammatoire de l'intestin ou d'une colite ischémique, la composition pharmaceutique comprenant un acide taurodésoxycholique ou un de ses sels pharmaceutiquement acceptables en tant que principe actif.

2. Composition pharmaceutique destinée à être utilisée selon la revendication 1, dans laquelle l'acide taurodésoxycholique est un composé représenté par la [formule chimique 1] suivante :

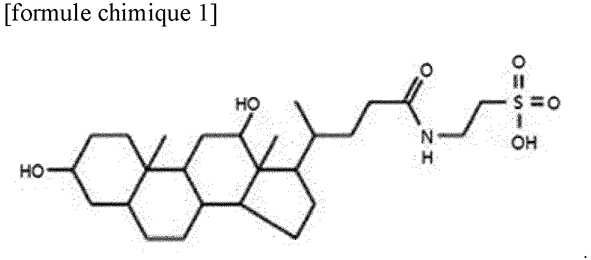

3. Composition pharmaceutique destinée à être utilisée selon la revendication 1, dans laquelle le sel pharmaceutiquement acceptable est un sel de sodium.

4. Composition pharmaceutique destinée à être utilisée selon la revendication 1, dans laquelle l'acide taurodésoxycholique ou un de ses sels pharmaceutiquement acceptables interdit une production de cytokines pro-inflammatoires.

5. Composition pharmaceutique destinée à être utilisée selon la revendication 4, dans laquelle les cytokines pro-inflammatoires sont un ou plusieurs éléments quelconques choisis dans le groupe constitué de l'IL-6, de l'IL-10 et du TNF-α.

6. Composition pharmaceutique destinée à être utilisée selon la revendication 1, dans laquelle l'acide taurodésoxycholique ou un de ses sels pharmaceutiquement acceptables diminue un nombre de cellules inflammatoires dans les intestins.

7. Composition pharmaceutique destinée à être utilisée selon la revendication 1, dans laquelle la maladie inflammatoire de l'intestin est sélectionnée parmi la colite ulcéreuse, la colite collagénique, la colite lymphoïde, la colite de diversion, la maladie de Crohn, le syndrome de Behcet et la colite indéterminée.

8. Aliment de santé destiné à être utilisé pour la prévention ou l'amélioration de la maladie inflammatoire de l'intestin ou d'une colite ischémique, l'aliment de santé comprenant un acide taurodésoxycholique ou un de ses sels pharmaceutiquement acceptables en tant que principe actif.

9. Aliment de santé destiné à être utilisé selon la revendication 8, dans lequel la maladie inflammatoire de l'intestin est sélectionnée parmi la colite ulcéreuse, la colite collagénique, la colite lymphoïde, la colite de diversion, la maladie de Crohn, le syndrome de Behcet et la colite indéterminée.
